# EUROPEAN PATENT APPLICATION

(11) **EP 4 446 141 A1**
(43) Date of publication of application: **16.10.2024**
(21) Application number: 24169120.3
(22) Date of filing: 09.04.2024
(51) Int. Cl.: B60H 1/00, G01N 33/00

(54) **VENTILATION SYSTEM COMPRISING A DETECTION ASSEMBLY FOR DETECTING AIR QUALITY IN A VEHICLE**

(30) Priority: 12.04.2023 IT 202300006972
(71) Applicant: DENSO THERMAL SYSTEMS S.p.A., 10046 Poirino (Torino) (IT)
(72) Inventor: CARLONE, Vito, 10046 POIRINO (Torino) (IT); VITALI, Domenico, 10046 POIRINO (Torino) (IT)
(74) Representative: Vanzini, Christian

(57) **Abstract**

Ventilation system comprising a detection assembly (30) for detecting air quality in a vehicle, the assembly (30) comprising an outdoor air duct (32) configured for outdoor air coming from an external environment to enter into the assembly (30), an indoor air duct (33) configured for indoor air coming from a cabin (C) of the vehicle to enter into the assembly (30), an air outlet (34) configured for the indoor air or outdoor air to exit from the assembly (30), assembly valve means (35) and at least one sensor (31) configured to provide a first signal indicative of the concentration of a pollutant in the outdoor air and a second signal indicative of the concentration of a pollutant in the indoor air, wherein the assembly valve means (35) are operable to expose the sensor (31), in a cyclic manner, alternatively to the outdoor air and to the indoor air, the sensor (31) providing alternatively, in a cyclic manner, the first signal and the second signal.

## Description

The present invention generally refers to vehicle ventilation and air conditioning systems.

The air quality within the cabin is very important for determining the health and well-being of the driver and passengers. Furthermore, as is known, driving performance is affected by the concentration of carbon dioxide within the cabin.

There is therefore a need for systems that are capable of automatically operating those flaps that are dedicated to controlling the entry of outdoor air from the external environment and the recirculation of air within the cabin.

One function of such systems is generally associated with controlling the concentration of CO₂ in the air within the cabin. Another function is aimed at preventing fogging of the windshield. A further function is aimed at preventing the introduction of pollutants originating from the external environment. An additional function is aimed as expelling odors or other undesirable substances present in the air within the cabin by replacing it with outdoor air originating from an air conditioning unit that is partially or fully set to outdoor air intake mode. Such functions are generally managed in having energy savings as the counter argument.

One problem that is felt is with regard to the intrinsic floating bias of some types of sensors that are dedicated to measuring the concentration of pollutants in the air, which requires a correction in order to enable the correct functioning of the system. Such correction takes place by zeroing the sensor under a condition wherein the pollutant is absent from the air. Unless such zeroing occurs in a controlled atmosphere, said condition, wherein the pollutant is absent from the air, is difficult to obtain.

One object of the invention is to provide a solution capable of at least partially obviating the aforementioned problem.

With respect to this object, the object of the invention is a vehicle ventilation system comprising
an outdoor air inlet configured for outdoor air coming from an external environment,
an indoor air inlet configured for indoor air coming from a cabin of the vehicle,
at least one blower connected with at least one of said outdoor air inlet and indoor air inlet and configured to generate an air flow intended to be introduced into the cabin of the vehicle through an outlet of the ventilation system,
ventilation control valve means interposed between at least one of said air inlets and said at least one blower and controllable to switch between an extreme outdoor air position, in which the outdoor air inlet is in fluid communication with the outlet of the ventilation system and the outlet of the ventilation system is fluidically shut off from the indoor air inlet, and an extreme indoor air position in which the indoor air inlet is in fluid communication with the outlet of the ventilation system and the outlet of the ventilation system is shut off from the outdoor air inlet, and
a detection assembly for detecting the quality of the air within the vehicle, said assembly comprising
an outdoor air duct connected to the outdoor air inlet and configured to tap the outdoor air from the outdoor air inlet,
an indoor air duct connected to the cabin and configured to tap the indoor air from the cabin,
an air outlet for the indoor air or outdoor air to exit from the assembly,
assembly valve means controllable to selectively open or close the outdoor air duct and correspondingly close or open the indoor air duct, and
at least one sensor interposed between the outdoor air duct and the indoor air duct and the air outlet, said at least one sensor being configured to provide a first signal indicative of the concentration of a pollutant in the outdoor air and a second signal indicative of the concentration of a pollutant in the indoor air,
wherein said assembly valve means are operable to expose said at least one sensor, in a cyclic manner, alternatively to the outdoor air and to the indoor air, said at least one sensor providing alternatively, in a cyclic manner, said first signal and second signal.

In equipping an air conditioning system or, more generally, a ventilation system with the aforementioned detection assembly, the system has measurements available thereto of the concentration of a certain pollutant in the outside and indoor air that are made using the same dedicated sensor. The rationale of the system may therefore operate on the basis of the comparison, or the difference between such measurements, without having to have a floating bias correction.

Other features and advantages of the system according to the invention will become apparent from the following detailed description of some embodiments of the invention, made with reference to the accompanying drawings, which are provided for illustrative and non-limiting purposes only, wherein:
- Figure 1 is a diagram representing an air conditioning system provided with a detection assembly according to the invention;
- Figure 2 is a diagram representing the detection assembly of the air conditioning system of Figure 1;
- Figure 3 is a diagram representing a variant of the detection assembly of Figure 2;
- Figures 4 and 5 are diagrams representing variants of a suction device of the detection assembly;
- the Figures from 6 to 9 are diagrams representing respective further embodiments of the air conditioning system;
- Figure 10 is a graph representing the work cycle of the detection assembly; and
- Figure 11 is a graph representing an example of a partial air quality index as a function of a generic pollutant.

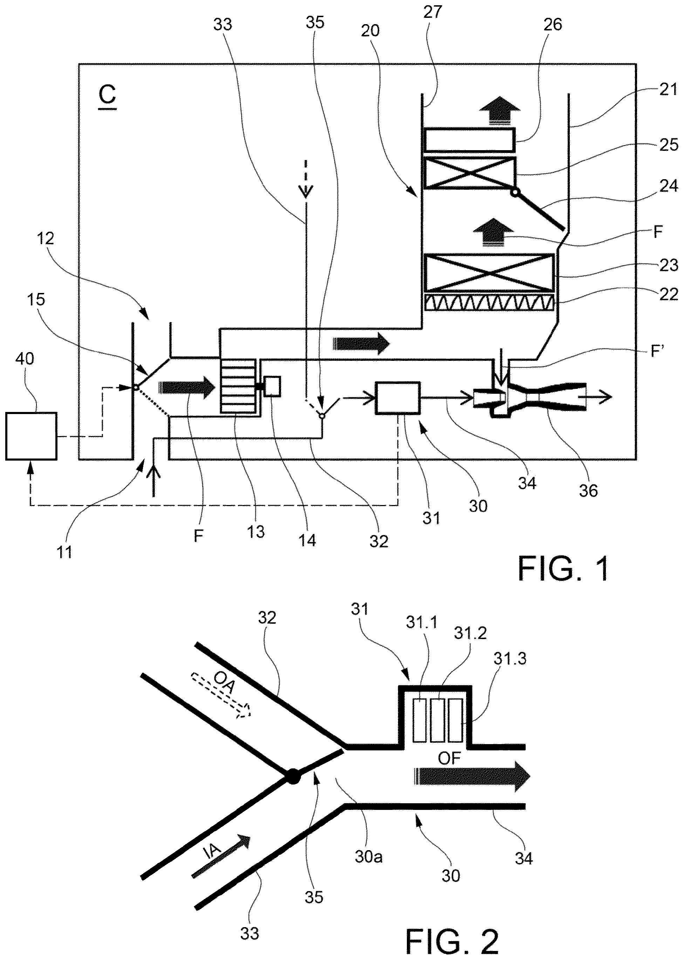

With reference to Figure 1, a vehicle air conditioning system comprises an outdoor air inlet 11 configured for air coming from an external environment to a cabin C of the vehicle, and an indoor air inlet 12 configured for air coming from the cabin C of the vehicle. Hereinafter air coming from the external environment will be referred to as outdoor air, whilst air coming from the cabin C of the vehicle will be referred to as indoor air. In place of the air conditioning system there may be a simple ventilation system which is not equipped for heating or dehumidifying air. In the description that follows reference will be made to an air conditioning system, but it is understood that the invention may be applied to a system that is dedicated to simple ventilation. Where not otherwise specified, the term "ventilation system" is therefore understood to include both pure ventilation and air conditioning systems.

The air conditioning system furthermore comprises a blower 13 connected to the outdoor air inlet 11 and to the indoor air inlet 12. The blower 13 is driven by a motor 14 in order to generate an air flow F intended to be introduced into the cabin C of the vehicle.

The air conditioning system furthermore comprises a ventilation control valve device 15 interposed between the air inlets 11, 12 and the blower 13 and controllable to switch between an extreme outdoor air position (represented by a continuous line in Figure 1), in which the outdoor air inlet 11 is in fluid communication with the blower 13 and the blower 13 is fluidically shut off from the indoor air inlet 12, and an extreme indoor air position (represented by a dotted line in Figure 1) in which the indoor air inlet 12 is in fluid communication with the blower 13 and the blower 13 is shut off from the outdoor air inlet 11. In the example shown, the ventilation control valve device 15 is implemented as a flap device, and in the following it will simply be indicated as such. According to one embodiment, the flap device 15 may switch to other intermediate positions between the extreme outdoor air position and the extreme indoor air position.

The flap device 15 is controlled by an actuator (not shown). In the example shown such device is represented as a single hinged flap, but it is understood that the flap device may comprise a plurality of flaps possibly with differing movements. The air conditioning system architecture or, more generally, the ventilation system may furthermore be different to that represented in the figure: for example it may include distinct sub-systems for outdoor air and for indoor air, provided with respective valve means and/or multiple blowers.

The air conditioning system represented in the figure furthermore comprises an air conditioning unit 20 envisaged for the conditioning of the air flow F intended to be introduced into the cabin C. In the case wherein instead of an air conditioning system there is a simple ventilation system such air conditioning unit would be absent. The air conditioning unit 20 comprises a casing 21, wherein there is arranged a filter 22 positioned downstream of the blower 13, an evaporator 23 configured to enable an exchange of heat between a refrigerant circulating in a circuit, not shown, and the air flow F, a flaps assembly 24 configured to orient/deflect the air flow F according to predetermined routes, an internal heat exchanger 25 and an electric heater 26, for example a PTC heater, which are configured so as to heat the air flow F. The architecture of the air conditioning unit 20 is not essential for the objectives of the invention and may be different than that represented in the figures. Generally indicated with the numeral 27 is an air conditioning system outlet, wherethrough the air flow F is introduced into the cabin C. Such outlet may generally comprise a plurality of outlets distributed within the cabin which are possibly equipped with respective closing/adjustment devices.

The air conditioning system furthermore comprises an assembly or detection assembly 30 branching off from the outdoor air inlet 11 upstream of the ventilation control valve means 15. The assembly or detection assembly 30 comprises a sensor block 31, which is formed from at least one sensor, and preferably from a plurality of sensors 31.1, 31.2, 31.3, wherein each thereof is a concentration sensor configured to provide a respective plurality of first signals and a respective plurality of second signals indicative of the concentration of respective pollutants in the outdoor air and in the indoor air. Possible examples of sensors comprise sensors that are capable of detecting particulate concentration, for example PM 2.5 or PM 10 sensors, or gas sensors such as for example for carbon monoxide (CO), carbon dioxide (CO₂), nitrogen oxides (NOₓ), ammonia (NH₃), volatile organic compounds (tVOCs), ozone (O₃), sulfur oxides (SOₓ), formaldehyde (CH₂O) and benzene (C₆H₆).

The air conditioning system and/or the detection assembly 30 comprise a control unit 40. The control unit 40 may be configured to control the flap device 15 based on signals supplied by the sensor block 31. The control unit 40 may furthermore be configured to control other components of the air conditioning system based on signals supplied by different sensors, but this is not essential to the objectives of the invention.

The detection assembly 30 is configured to expose the sensors 31.1, 31.2, 31.3 (or a part thereof), in a cyclical manner, alternatively to the outdoor air and to the indoor air, in such a way that each of the sensors 31.1, 31.2, 31.3 (or a part thereof) provides, in a cyclical manner, a first signal indicative of the concentration of a pollutant in the outdoor air and a second signal indicative of the concentration of a pollutant in the indoor air.

To this end, the detection assembly 30 furthermore comprises an outdoor air duct 32 and an indoor air duct 33 wherethrough, respectively, the outdoor air and the indoor air enter into the detection assembly 30, an air outlet 34 wherethrough the outdoor air or the indoor air exit from the detection assembly 30, and a valve assembly 35 that is controllable to selectively open or close the outdoor air duct 32 and correspondingly close or open the indoor air duct 33. The outdoor air duct 32 has an inlet that is connected to the outdoor air inlet 11 of the air conditioning system for purging outdoor air therefrom, whilst the indoor air duct 33 has an inlet that is connected to the inside of the cabin C for purging the indoor air from the cabin C. The sensors 31.1, 31.2, 31.3 (or at least a part thereof) are interposed between the outdoor air duct 32 and the indoor air duct 33 and the air outlet 34. For the detection assembly 30 to be capable of drawing outside or indoor air through the respective inlets, positioned upon the air outlet 34 of the detection assembly 30 is a suction device 36 configured to generate an air flow that passes within the sensor block 31 of the detection assembly 30. In the example shown in Figure 1, the suction device 36 is implemented as a Venturi ejector that uses, as a working fluid, a part of the air flow F generated by the blower 13 of the air conditioning system, indicated with the letter F' in Figure 1. To such end, the lateral inlet of the ejector is connected to the air conditioning unit 20 upstream of the filter 22. In the example represented in Figure 1, the air outlet 34 discharges the air leaving the detection assembly 30 into the cabin C.

Represented in Figure 2 is a possible configuration of the detection assembly 30, wherein the valve assembly 35 is implemented as a single valve arranged at the confluence 30a between the outdoor air duct 32 and the indoor air duct 33. The sensor block 31 is arranged downstream of the confluence 30a between the outdoor air duct 32 and the indoor air duct 33. The arrows OA and IA represent, respectively, the outdoor air flow and the indoor air flow which pass within the respective ducts, whilst the arrow OF represents the outlet air flow directed towards the suction device 36. Such outlet air flow OF coincides with the outdoor air flow OA or with the indoor air flow IA according to the position of the valve assembly 35.

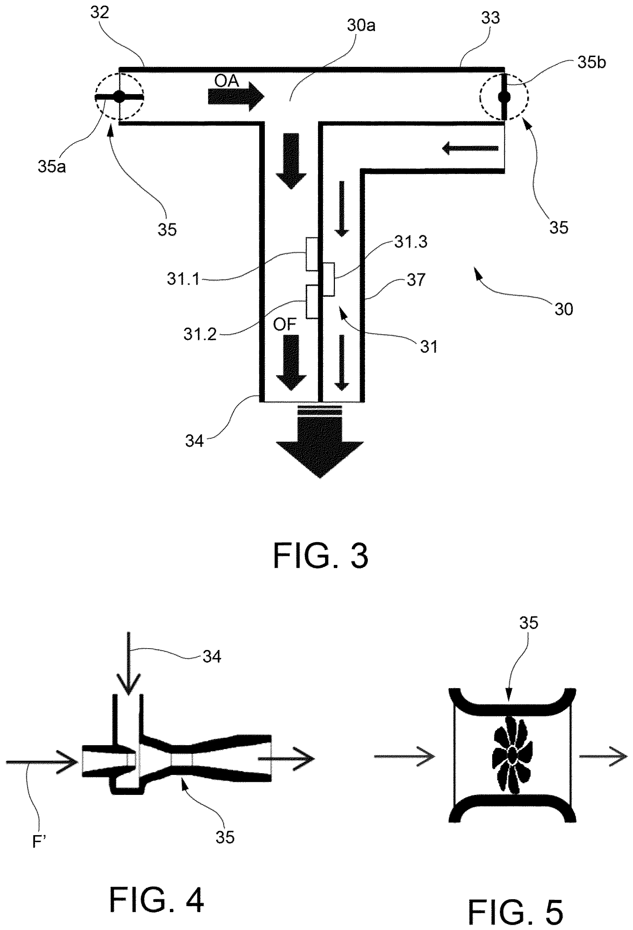

Represented in Figure 3 is a further possible configuration of the detection assembly 30, wherein the valve assembly 35 is implemented as a pair of valves 35a and 35b respectively arranged along the outdoor air duct 32 and along the indoor air duct 33, upstream of the confluence 30a. The sensor block 31 is arranged downstream of the confluence 30a between the outdoor air duct 32 and the indoor air duct 33. The arrow OA represents, respectively, the outdoor air flow that passes within the respective duct, whilst the indoor air flow is not represented in Figure 3 since the respective valve 35b is shown to be closed. The arrow OF represents the outlet air flow directed towards the suction device 36. Such outlet air flow OF coincides with the outdoor air flow OA or with the indoor air flow according to the position of the valve assembly 35. Further represented in Figure 3 is a secondary indoor air duct 37, having an inlet connected to the inside of the cabin C and an outlet connected to the suction device 36. Such secondary indoor air duct 37 may be provided whenever one or more of the sensors 31.1, 31.2, 31.3 (for example a CO₂ concentration sensor) is dedicated to detecting the concentration of the respective pollutant only in the indoor air. For such sensor the cyclical detection logic discussed within the present description is not applied.

Represented in Figure 4 is the detail of the suction device 36 according to one variant, according to which the suction device 36 is again implemented as a Venturi ejector that uses, as a working fluid, a part of the air flow F generated by the blower 13 of the air conditioning system. Such part of the air flow F' is however introduced into a central inlet of the ejector that is connected to the air conditioning unit 20 upstream of the filter 22.

Represented in Figure 5 is the detail of the suction device 35 according to a further variant which envisages that the suction device 35 be implemented as a blower. Such solution requires that there be a motor for driving the blower 35, whilst in the variants of Figures 1 and 4 the Venturi effect suction device is simply driven by the relative working fluid.

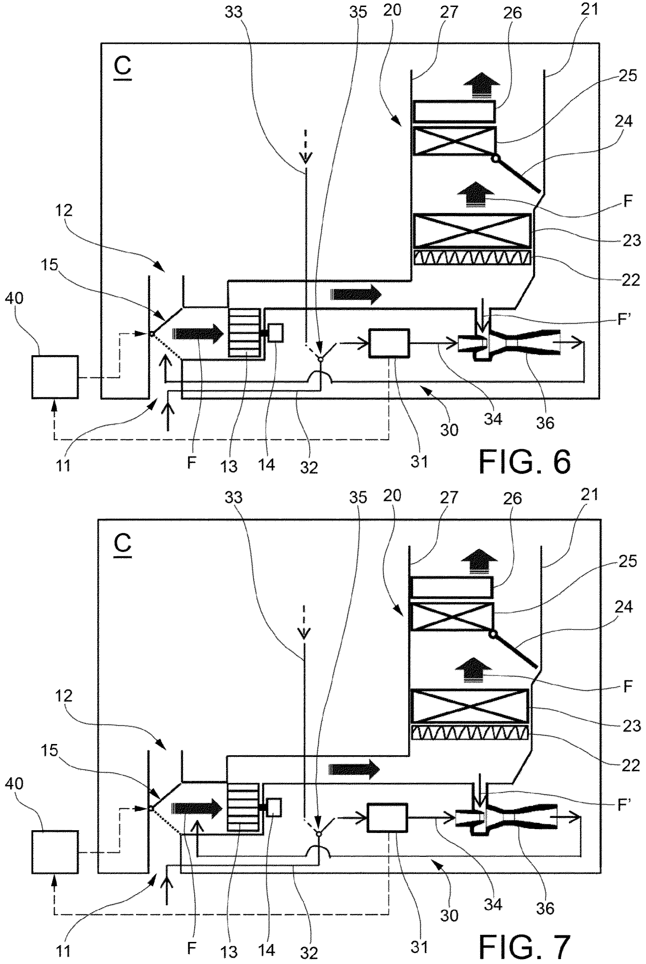

Represented in Figure 6 is a further embodiment of the air conditioning system. Elements corresponding to those of the foregoing embodiment have been assigned the same numerical references. These elements will not be further described.

Such embodiment differs from the embodiment in Figure 1 by the fact that the outlet of the detection assembly 30 (discharge side of the suction device 36) is connected to the fresh air inlet 11 of the air conditioning system, downstream of the inlet of the outdoor air duct 32 of the detection assembly 30, but upstream of the flap device 15. Such embodiment, when the flow of outdoor air passes within the detection assembly 30, no matter how small it is, prevents the flow from directly arriving at the cabin C without having been treated by the air conditioning unit 20.

Represented in Figure 7 is a further embodiment of the air conditioning system. Elements corresponding to those of the embodiment of Figure 1 have been assigned the same numerical references. These elements will not be further described.

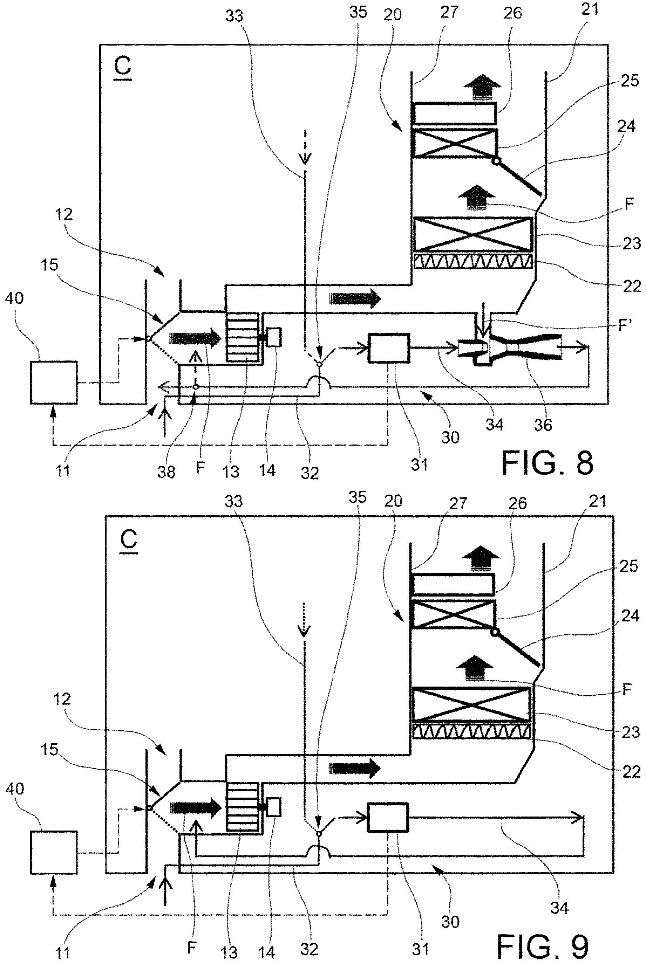

Such embodiment differs from the embodiment in Figure 1 by the fact that the outlet of the detection assembly 30 (discharge side of the suction device 36) is connected to the suction side of the blower 13 of the air conditioning system, downstream of the flap device 15. Such embodiment, when the flow of outdoor air passes within the detection assembly 30, no matter how small it is, prevents the flow from directly arriving at the cabin C without having been treated by the air conditioning unit 20. Furthermore, such embodiment prevents the flow of air discharged from the detection assembly 30 from accumulating at the outdoor air inlet 11 of the air conditioning system when the flap device 15 closes the outdoor air inlet 11, as may happen in the embodiment of Figure 6. A variant of this configuration is represented in Figure 9. Such variant is devoid of a dedicated suction device, and the air outlet 34 of the detection assembly 30 is connected directly to the suction side of the blower 13. This is possible in the case wherein the suction at the blower 13 inlet is sufficient to generate the flow of air required in order to pass through the sensor block 31. This configuration has the advantage of being simpler and more economical insofar as there is one component fewer (the ejector or other dedicated device), and it is also more efficient in that it does not lead to losses of air from the conditioning unit (F'=0).

Represented in Figure 8 is a further embodiment of the air conditioning system. Elements corresponding to those of the embodiment of Figure 1 have been assigned the same numerical references. These elements will not be further described.

Such embodiment differs from the embodiment in Figure 1 by the fact that the outlet of the detection assembly 30 (discharge side of the suction device 36) comprises an outlet valve assembly 38 that has a first outlet connected to the fresh air inlet 11 of the air conditioning system, downstream of the inlet of the outdoor air duct 32 of the detection assembly 30, and a second outlet connected to the suction side of the blower 13 of the air conditioning system, downstream of the flap device 15. Compared to the embodiments of Figures 6 and 7, such embodiment makes it possible to manage the discharging of the detection system 30 as a function of the operational state of the flap device 15. For example, the control unit 40 may control the outlet valve means 38 in such a way as to place the outlet of the detection assembly 30 in communication with the first outlet of the outlet valve means, and correspondingly shut off the outlet of the detection assembly 30 from the second outlet of the outlet valve means 38, when the flap device 15 is in the extreme outdoor air position, and to place the outlet of the detection assembly 30 in communication with the second outlet of the outlet valve means, and correspondingly to shut off the outlet of the detection assembly 30 from the first outlet of the detection assembly 38 when the flap device 15 is in the extreme indoor air position.

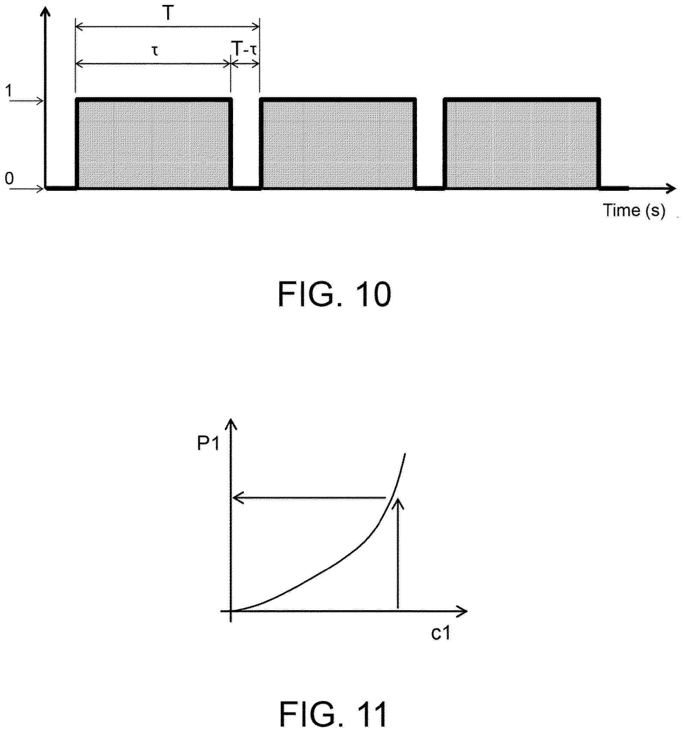

Represented in the Figure 10 is the work cycle of the detection assembly 30. In such graph, time is represented on the x-axis and the operational state of the detection assembly is represented on the y-axis. Represented with "0" is the state wherein the detection assembly 30 exposes the sensors 31.1, 31.2, 31.3 (or a subassembly thereof) to the indoor air, and such sensors provide the respective second signals indicative of the concentration of the respective pollutants in the indoor air. Represented with "1" is the state wherein the detection assembly 30 exposes the sensors 31.1, 31.2, 31.3 (or a subassembly thereof) to the outdoor air, and such sensors provide the respective first signals indicative of the concentration of the respective pollutants in the outdoor air. The work cycle period is indicated with T, which may assume for example, values within the range 60 s ≤ T ≤ 600 s. Low values are preferable in order to increase the frequency of oscillation between outdoor air and indoor air analysis. The period of time wherein the operational state of the detection assembly assumes the value 1 is indicated with τ (namely the period of time period wherein the detection assembly performs the outdoor air analysis). It is generally preferable for the detection assembly to be predominantly in the 1 state, or at most in the two states for the same quantity of time, i.e., 50% ≤ τ/T < 100%.

Advantageously, the functional logic of the control unit 40 is based on a calculation of an aggregate index of the outdoor air quality and a calculation of an aggregate index of the indoor air quality.

With reference to the following table:

| Type of pollutant | Indoor air concentration | Outdoor air concentration | Partial indoor AQI | Partial outdoor AQI |
|---|---|---|---|---|
| Pollutant #1 | c₁ | d₁ | P₁(c₁) | Q₁(d₁) |
| Pollutant #2 | c₂ | d₂ | P₂(c₂) | Q₂(d₂) |
| Pollutant #3 | c₃ | d₃ | P₃(c₃) | Q₃(d₃) |
| Pollutant #4 | c₄ | d₄ | P₄(c₄) | Q₄(d₄) |
| Pollutant #5 | c₅ | d₅ | P₅(c₅) | Q₅(d₅) |

The magnitude cₚ represents the concentration of the p^{th} pollutant in the indoor air (with the index p varying from 1 to N, where N is the number of pollutants considered), the magnitude dₚ represents the concentration of the p^{th} pollutant in the outdoor air, the magnitude Pₚ(cₚ) represents the partial index of the indoor air quality of the p^{th} pollutant, as a function of the concentration of the p^{th} pollutant in the indoor air, and the magnitude Qₚ(dₚ) represents the partial index of the outdoor air quality of the p^{th} pollutant, as a function of the concentration of the p^{th} pollutant in the outdoor air. Represented in Figure 11, merely as an example, is the possible trend of the partial index of the indoor air quality P₁ as a function of the concentration c₁ of the pollutant #1 in the indoor air. Naturally, this is only an example. In general, a partial index air quality curve is a monotonic (not strictly) increasing curve, generally nonlinear, with an increasing slope as the concentration of the pollutant increases, possibly with sections that are less steep.

Known to a person skilled in the art are various formulas that exist in the literature and which express air quality index in relation to a pollutant datum. The present invention is not however limited to a specific formula.

In general, it may be assumed that the functions Qₚ express values that are greater compared to the respective Pₚ functions, at the same concentration of the p^{th} pollutant, since the possible entry of harmful pollutants into the cabin depends upon the presence of such pollutants in the outdoor air and, most importantly, the dangerousness of a pollutant coming from the outside is in general greater than the dangerousness of a pollutant coming from inside the cabin.

To give an example in the case of measuring odors using tVOC sensors, those odors that are generated inside the cabin may generally be associated with substances that are much less dangerous than those coming from the external environment in that the latter may, for example, derive from vehicle exhaust gases.

The control unit 40 may calculate an aggregate indoor air quality index AQI_{indoor} as a function of the partial indoor air quality indices relating to the various pollutants, AQI_{indoor}=F(P₁, ..., P_{N}), and an aggregate outdoor air quality index AQI_{outdoor} as a function of the partial indoor air quality indices relating to the various pollutants, AQI_{outdoor}=G(Q₁, ..., Q_{N}). For example, the index AQI_{indoor} may be given by a datum of a (possibly normalized) summation of the partial indoor air quality indices relating to the various pollutants, and the index AQI_{outdoor} may be given by a datum of a (possibly normalized) summation of the partial outdoor air quality indices relating to the various pollutants.

In particular, the control unit 40 is configured to
calculate an aggregate outdoor air quality index based on the concentration values of the outdoor air pollutants provided by the sensors 31.1, 31.2, 31.3 and an aggregate indoor air quality index based on the concentration values of the indoor air pollutants provided by the sensors 31.1, 31.2, 31.3 and
compare the aggregate outdoor air quality index with the aggregate indoor air quality index, and
control the flap device 15 based on such comparison.

The control logic based on the calculation of the air quality indices obtains an important advantage from the fact that each pollutant is measured with the respective sensor, independently of the fact that it is contained within outdoor or indoor air. This makes it possible to have reliable measurements that are not affected by the issues of conventional systems.

Merely by way of illustrative example a possible automatic actuation sequence for a flap device 15 will now be described.

The flap device 15 is initially found to be in the extreme outdoor air position. Such condition is maintained until the AQI_{outdoor} index is better than the AQI_{indoor} index. During such step the detection assembly 30 work cycle is set so that the period τ is set to the maximum possible, and the period t-τ during which the detection assembly is found to be in the 0 state is set to the minimum possible but still sufficient to enable the sensors to measure the concentrations of pollutants in the indoor air in a reliable manner. When the AQI_{outdoor} index becomes worse than the AQI_{indoor} index (for example when the vehicle enters into a tunnel) the flap device 15 is switched over. Alternatively, such switching may be activated by the detection of an energy consumption condition that is greater than a certain threshold. In addition other logical conditions may be considered to be managed, if they enter into competition therebetween, using different priorities.

Following the detection of a worsening of the outdoor air quality the flap device 15 is switched to the extreme indoor air position thereby implementing a total recirculation condition. Subsequently, following a relative improvement in outdoor air quality, compared to the indoor air quality, based also upon other functional logic, the flap device 11 may be moved to an intermediate position between the extreme outdoor air position and the extreme indoor air position, therefore enabling the circulation of recirculation air with a variable ratio between the recirculating air flow F and the fresh air flow. During such step the detection assembly 30 work cycle is set so that the period τ is varied so as to favor the reading of indoor air for longer, given that it is indoor air that enters the air conditioning unit during this step, nonetheless monitoring the outdoor air in order to enable the selection of the next step at the right point in time, as described hereinafter. Such condition is maintained until the AQI_{indoor} index is better than the AQI_{outdoor} index. When the AQI_{indoor} index becomes worse than the AQI_{outdoor} index the flap device 15 is switched to the extreme outdoor air position. Alternatively, the switch from the extreme indoor air position to the extreme outdoor air position may be activated by the detection of a windshield fogging condition. Such method, described for outdoor air, may also be partial, maintaining the flap device 15 at an intermediate position that is between the extreme positions.

## Claims

1. Vehicle ventilation system, comprising
an outdoor air inlet (11) configured for air coming from an external environment,
an indoor air inlet 12 configured for air coming from the cabin C of the vehicle,
at least one blower (13) connected to at least one of said outdoor air inlet (11) and indoor air inlet (12) and configured to generate an air flow (F) intended to be introduced into the cabin (C) of the vehicle through an outlet (27) of the ventilation system,
ventilation control valve means (15) interposed between at least one of said air inlets (11, 12) and said at least one blower (13) and controllable to switch between an extreme outdoor air position, in which the outdoor air inlet (11) is in fluid communication with the outlet (27) of the ventilation system and the outlet (27) of the ventilation system is fluidically shut off from the indoor air inlet (12), and an extreme indoor air position in which the indoor air inlet (12) is in fluid communication with the outlet (27) of the ventilation system and the outlet (27) of the ventilation system is shut off from the outdoor air inlet (11), and
a detection assembly (30) for detecting air quality in the vehicle,
the ventilation system being **characterized in that** the detection assembly comprises
an outdoor air duct (32) connected to the outdoor air inlet (11) and configured to tap the outdoor air from the outdoor air inlet (11),
an indoor air duct (33) connected to the cabin (C) and configured to tap the indoor air from the cabin (C),
an air outlet (34) configured for the indoor air or outdoor air to exit from the detector assembly (30),
assembly valve means (35) controllable to selectively open or close the outdoor air duct (32) and correspondingly close or open the indoor air duct (33), and
at least one sensor (31.1, 31.2, 31.3) interposed between the outdoor air duct (32) and the indoor air duct (33) and the air outlet (34), said at least one sensor (31.1, 31.2, 31.3) being configured to provide a first signal indicative of the concentration of a pollutant in the outdoor air and a second signal indicative of the concentration of a pollutant in the indoor air,
wherein said assembly valve means (35) are operable to expose said at least one sensor, in a cyclic manner, alternatively to the outdoor air and to the indoor air, said at least one sensor providing alternatively, in a cyclic manner, said first signal and second signal.

2. Ventilation system according to claim 1, wherein said at least one sensor (31.1, 31.2, 31.3) is arranged downstream or at an area (30a) whereinto the outdoor air duct (32) and the indoor air duct (33) flow.

3. Ventilation system according to claims 1 or 2, furthermore comprising a suction device (36) operable to suck air in the assembly (30) through the outdoor air duct (32) or the indoor air duct (33).

4. Ventilation system according to claims 3, wherein the suction device (36) comprises a Venturi ejector configured to use, as a working fluid, part (F') of the air flow (F) generated by a blower of a ventilation system.

5. Ventilation system according to claims 1 or 2, wherein the air outlet (34) is configured to connected the suction side of a blower of a ventilation system.

6. Ventilation system according to any one of the preceding claims, wherein the air outlet (34) of the detection assembly (30) is configured to discharge air directly into the cabin (C).

7. Ventilation system according to any one of the claims from 1 to 5, wherein the air outlet (34) of the detection assembly (30) is connected to the outdoor air inlet (11) of the ventilation system, upstream of the ventilation control valve means (15), or to the suction side of the blower (13), downstream of the ventilation control valve means (15).

8. Ventilation system according to any one of the preceding claims, wherein said at least one sensor comprises a plurality of sensors (31.1, 31.2, 31.3) configured to provide a respective plurality of first signals and a respective plurality of second signals indicative of the concentration of respective pollutants in the outdoor air and in the indoor air,
wherein the detection assembly furthermore comprises a control unit (40) configured to calculate an aggregate outdoor air quality index based on said plurality of fist signals and an aggregate indoor air quality index based on said plurality of second signals, wherein in cui the aggregate outdoor air quality index and the aggregate indoor air quality index are a function of a plurality of partial air quality indices relating to respective pollutants, respectively of the outdoor air and of the indoor air.

9. Ventilation system according to claim 8, wherein the control unit (40) is configured to
compare the aggregate outdoor air quality index with the aggregate indoor air quality index, and
control the ventilation control valve means (15) based on said comparison.
